# EUROPEAN PATENT SPECIFICATION

(11) **EP 0 613 459 B1**
(45) Date of publication and mention of the grant of the patent: **06.03.1996**
(21) Application number: 92921774.3
(22) Date of filing: 13.10.1992
(51) Int. Cl.: C07C 229/24, C07F 9/40, C07F 9/38

(54) **N-TRITYL ASPARTIC ACID DERIVATIVES FOR PREPARING PHOSPHONATE NMDA ANTAGONISTS**
N-TRITYL ASPARAGINSÄUREDERIVATE ZUR HERSTELLUNG VON PHOSPHONAT NMDA ANTAGONISTEN
DERIVES D'ACIDE N-TRITYLE ASPARTIQUE UTILISES POUR LA PREPARATION D'ANTAGONISTES DE PHOSPHONATE NMDA

(30) Priority: 15.11.1991 US 792834
(43) Date of publication of application: 07.09.1994
(73) Proprietor: MERRELL DOW PHARMACEUTICALS INC., Cincinnati Ohio 45215-6300 (US)
(72) Inventor: WHITTEN, Jeffrey, P., Cincinnati, OH 45241 (US); RUDISILL, Duane, E., Indianapolis, IN 46268 (US)
(74) Representative: VOSSIUS & PARTNER
(86) International application number: US9208739
(87) International publication number: WO9310075

(56) References cited:
- EP-A- 0 418 863
- TETRAHEDRON, (INCL. TETRAHEDRON REPORTS) vol. 44, no. 2, 1988, OXFORD GB pages 637 - 642 JACK E. BALDWIN 'Synthesis and rearrangement of homoserine derivatives'

## Description

The present invention is related to novel (R)-dimethyl N-tritylaspartate and novel (R)-alkyl N-trityl-4-oxo-5-phosphononorvalinate derivatives which are useful in the preparation of a class of beta-ketophosphonate NMDA antagonists.

The class of beta-ketophosphonate NMDA antagonists is important in the treatment of epilepsy, nerve trauma such as that caused by stroke, cardiac arrest, hypoglycemia, and physical damage to either the brain or spinal cord, neurogenerative diseases, anxiety and for the relief of pain [European Application No. 91107955.6 of Jeffrey P. Whitten entitled Heterocyclic-NMDA Antagonists, filed May 16, 1991]. This class of excitatory amino acid antagonists which act at the NMDA receptor complex can be described by formula I wherein R is hydrogen, methyl or ethyl:

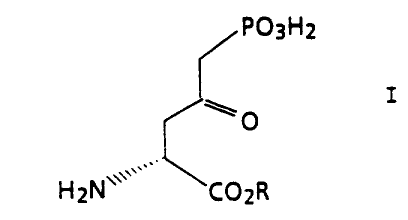

(R)-4-Oxo-5-phosphononorvaline derivatives of formula I have been synthesized by protecting (R)-aspartic acid as the CBZ oxazolone, converting the resulting acid to an acid chloride and then coupling with a cuprate to form the beta-ketophosphonate. A two step deprotection yielded the desired (R)-4-oxo-5-phosphononorvaline derivative of formula I after chromatography.

In EP-A 4 188 63 beta ketophosphonate NMDA antagonists are disclosed. According to this document the synthesis of these compounds requires the step of protecting the free amino group of an aminoacid or derivative with a steroyl carbamate protecting group.

The novel (R)-dialkyl N-tritylaspartates and novel (R)-alkyl N-trityl-4-oxo-5-phosphononorvalinate derivatives of the present invention are useful in the preparation of the class of beta-ketophosphonate NMDA antagonists. These compounds afford the (R)-4-oxo-5-phosphononorvaline derivatives of formula I with a high retention of chirality from readily available (R)-aspartic acid. In addition, the use of the novel (R)-dialkyl N-tritylaspartates and novel (R)-alkyl N-trityl-4-oxo-5-phosphononorvalinate derivatives of the present invention allows for a shorter synthesis with higher yields and avoids the use of copper. Further, the use of (R)-dimethyl N-tritylaspartate and (R)-methyl N-trityl-4-oxo-5-phosphononorvalinate derivatives of the present invention provides stable crystalline materials at every step thereby avoiding the necessity of chromatography.

The present invention provides novel (R)-dialkyl N-tritylaspartates of formula II and novel (R)-alkyl N-trityl-4-oxo-5-phosphonomervalinate derivatives of formula III, 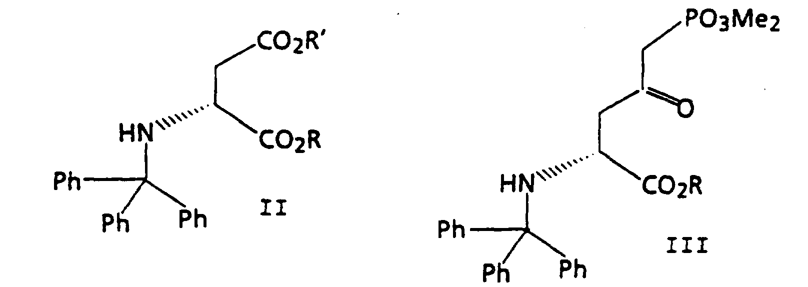 wherein R and R' are simultaneously methyl or ethyl.

The present invention further provides a method of using a (R)-dialkyl N-tritylaspartate of formula II comprising the steps of (a) reacting the (R)-dialkyl N-tritylaspartate of formula II with an appropriate metallo dimethyl methylphosphonate to give a (R)-alkyl N-trityl-4-oxo-5-phosphononorvalinate derivative of formula III and (b) reacting said (R)-alkyl N-trityl-4-oxo-5-phosphononorvalinate derivative of formula III with a suitable aqueous acid or trimethylsilyl iodide. It is preferred that R and R' are simultaneously methyl. As used herein, Ph represents an unsubstituted phenyl ring.

The present invention further provides a process for preparing the (R)-4-oxo-5-phosphononorvaline derivatives of formula I comprising the steps of (a) reacting a (R)-dialkyl N-tritylaspartate of formula II with an appropriate metalio dimethyl methylphosphonate to give a (R)-alkyl N-trityl-4-oxo-5-phosphononorvalinate derivative of formula III and (b) reacting said (R)-alkyl N-trityl-4-oxo-5-phosphononorvalinate derivative of formula III with a suitable acid or a suitable trimethylsilyl halide. It is preferred that R and R' are simultaneously methyl.

The novel (R)-dialkyl N-tritylaspartates of formula II and novel (R)-alkyl N-trityl-4-oxo-5-phosphononorvalinate derivatives of formula III can be prepared by utilizing procedures and techniques well known and appreciated by one of ordinary skill in the art. A general synthetic procedure for preparing these compounds is set forth in Scheme A.

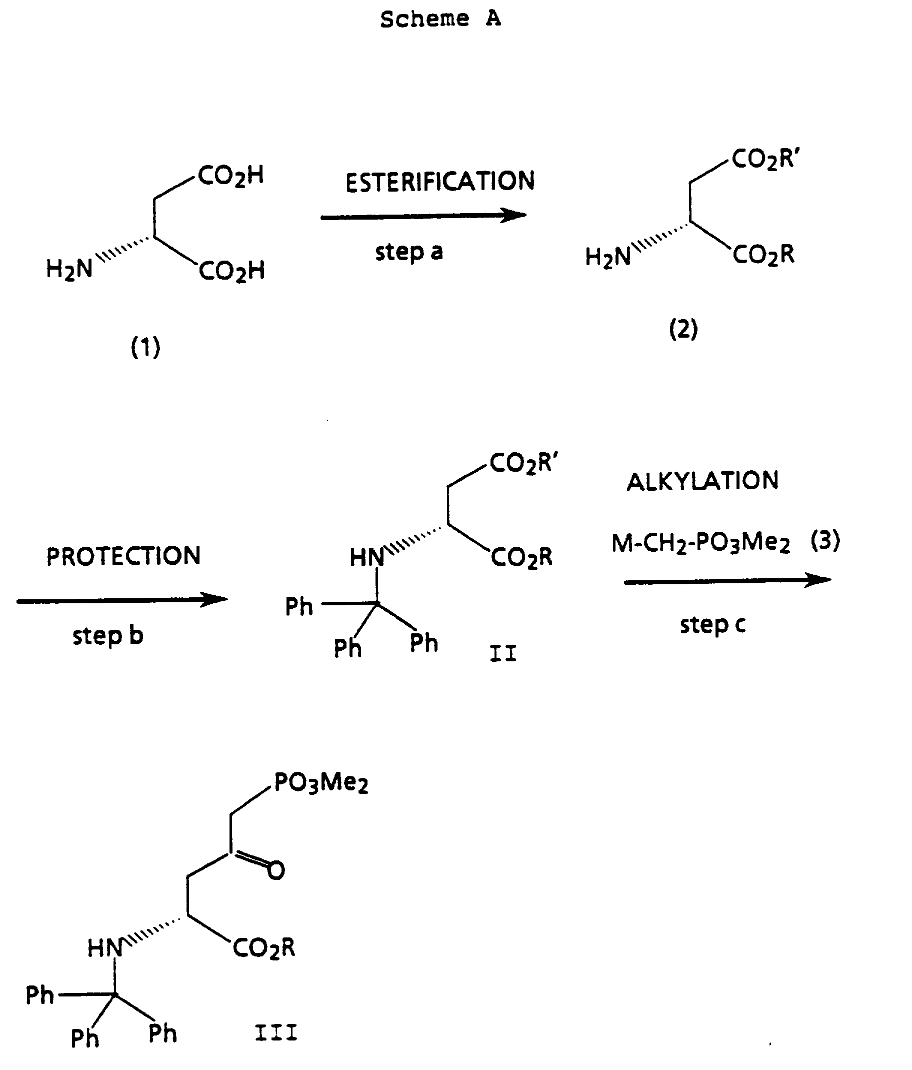

Scheme A provides a synthetic procedure for preparing (R)-dialkyl N-tritylaspartates of formula II and novel (R)-alkyl N-trityl-4-oxo-5-phosphononorvalinate derivatives of formula III.

In step a, (R)-aspartic acid (1) is esterified to give (R)-dialkylaspartate (2) by techniques and procedures well known and appreciated by one of ordinary skill in the art. For example, (R)-aspartic acid (1) is converted to (R)-dialkylaspartate (2) by adding a suitable activating agent, such as thionyl chloride, to a suspension of the (R)-aspartic acid (1) in a suitable alcohol such as methanol or ethanol. The reactants are typically stirred together for a period of time ranging from 10 hours to 3 days and at a temperature range of from 0°C to room temperature. The resulting (R)-dialkylaspartate (2) is recovered from the reaction zone by evaporation of the solvent.

In step b, (R)-dialkylaspartate hydrochloride (2) is converted to the (R)-dialkyl N-tritylaspartate of formula II by treating (R)-dialkylaspartate hydrochloride (2) with triphenylmethyl chloride and a suitable base, such as triethylamine. The reactants are typically contacted in a suitable organic solvent such as acetonitrile. The reactants are typically stirred together at room temperature for a period of time ranging from 2-24 hours. The resulting (R)-dialkyl N-tritylaspartate of formula II is recovered from the reaction zone by extractive methods as is known in the art.

In step c, the (R)-dialkyl N-tritylaspartate of formula II is converted to the (R)-alkyl N-trityl-4-oxo-5-phosphononorvalinate derivative of formula III by treating the (R)-dialkyl N-tritylaspartate of formula II with a suitable metallo methyl dimethylphosphonate of structure (3). Suitable metallo compounds of structure (3) include lithium methyl dimethylphosphonate, potassium methyl dimethylphosphonate, sodium methyl dimethylphosphonate, magnesium methyl dimethylphosphonate and the like. The reactants are typically contacted in a suitable anhydrous organic solvent such as tetrahydrofuran. The reactants are typically stirred together for a period of time ranging from 15 minutes to 5 hours and at a temperature range of from -78°C to -40°C. The (R)-alkyl N-trityl-4-oxo-5-phosphononorvalinate derivative of formula III is recovered from the reaction zone by acid neutralization followed by extractive methods as is known in the art. It may be purified by recrystallization.

Starting materials for use in Scheme A are readily available to one of ordinary skill in the art.

The following examples present typical syntheses as described in Scheme A. These examples are understood to be illustrative only and are not intended to limit the scope of the present invention in any way. As used herein, the following terms have the indicated meanings: "g" refers to grams; "mmol" refers to millimoles; "mL" refers to milliliters; "bp" refers to boiling point; "mp" refers to melting point; "°C" refers to degrees Celsius; "mm Hg" refers to millimeters of mercury; "µL" refers to microliters; "µg" refers to micrograms; and "µM" refers to micromolar.

### Example 1

### (R)-Dimethyl N-tritylaspartate

### Step a: (R)-Dimethylaspartate hydrochloride

Suspend (R)-aspartic acid (39.9g, 0.30mol) in methanol (250mL), cool to 0°C and place under a nitrogen atmosphere. Add, by dropwise addition over 0.5 hours, thionyl chloride (50.lg, 0.42mol). Allow to warm to room temperature and stir for 48 hours. Evaporate the solvent, triturate the residue with ether and collect by filtration to give the title compound as a white solid (60.1g, 100%).

¹H NMR (DMSO-d₆) δ 4.3 (t, 1H), 3.75 (s, 3H), 3.67 (m, 2H).

### Step b: (R)-Dimethyl N-tritylaspartate

Mix (R)-dimethyl aspartate hydrochloride (57g, 0.288mol), triphenylmethyl chloride (75.0g, 0.27mol) and acetonitrile. Stir well and add, by dropwise addition over 2 hours, triethylamine (85mL, 0.27mol). Stir the resulting mixture for a further 4 hours then add ethyl acetate (500mL). Wash with saturated sodium chloride (500mL) and add chloroform (100mL). Separate the organic phase, dry (Na₂SO₄) and evaporate to a residue. Add methanol (250mL) and stir to effect crystallization. Filter to give the title compound as a white solid (83g, 74%).

¹H NMR (CDCl₃) δ 7.5-7.25 (m, 15H), 4.78 (m, 1H), 4.72 (s, 3H), 3.3 (s, 3H), 2.95 (bs, 1H), 2.62 (m, 2H).

### Example 2

### (R)-Methyl N-trityl-4-oxo-5-(dimethylphosphono)norvalinate

Dissolve dimethyl methylphosphonate (83mL, 0.766mol) in anhydrous tetrahydrofuran (800mL), cool to -78°C and place under an inert atmosphere. Add, by dropwise addition over 15 minutes, n-butyllithium (306mL of a 2.5M solution, 0.766mol). Stir the resulting clear solution for a further 0.5 hour. While maintaining the solution at -78°C, add by dropwise addition over 15 minutes, to another solution of (R)-dimethyl N-tritylaspartate (71g, 0.182mol) in anhydrous tetrahydrofuran (200mL) also at -78°C. Stir for a further 0.5 hours, quench with acetic acid (45mL) and warm to room temperature. Add ethyl acetate (500mL) and saturated sodium bicarbonate (250mL). Separate the organic phase, filter through silica gel and evaporate the solvent to give a solid residue. Crystallize (t-butylmethyl ether/hexane) to give the title compound as a white solid (61.0g, 69%).

¹H NMR (CDCl₃) δ 7.5-7.25 (m, 15H), 3.82 (s, 3H), 3.79 (s, 3H), 3.75 (t, 3H), 3.08 (d, 2H), 2.88 (m, 2H), 1.65 (bs, 1H).

The (R)-4-oxo-5-phosphononorvaline derivatives of formula I can be prepared from the (R)-alkyl N-trityl-4-oxo-5-phosphononorvalinate derivative of formula III as set forth in Scheme B.

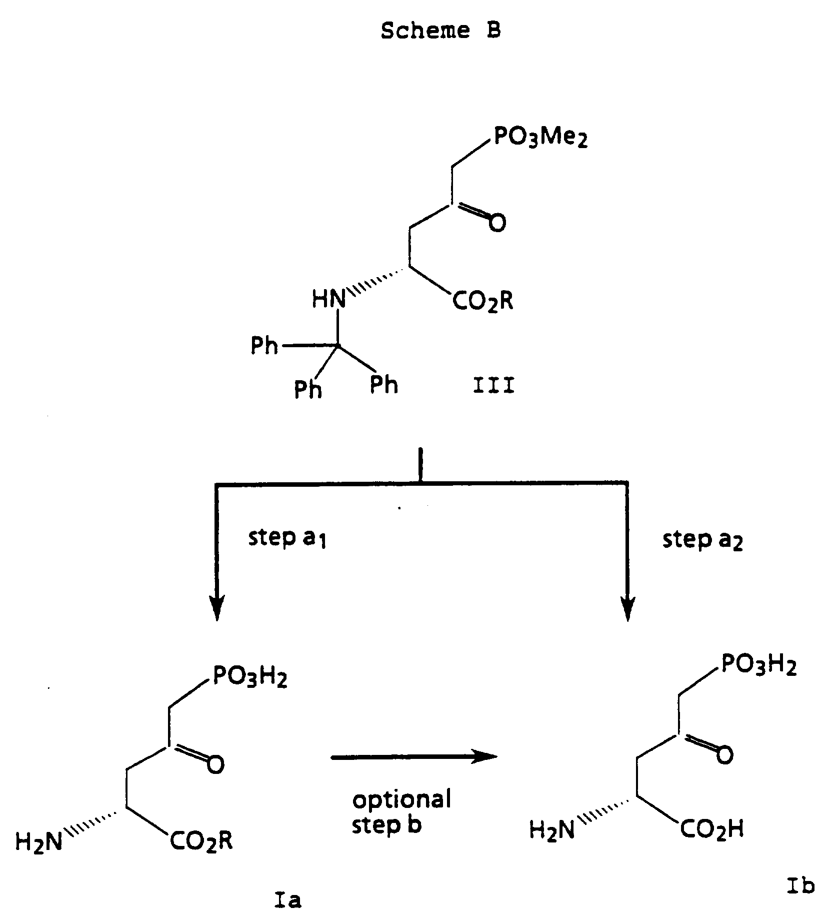

Scheme B provides a general method for using the (R)-alkyl N-trityl-4-oxo-5-phosphononorvalinate derivatives of formula III in order to form the (R)-4-oxo-5-phosphononorvaline derivatives of formula I.

In step a₁, the phosphonate methyl ester functionalities of the appropriate (R)-alkyl N-trityl-4-oxo-5-phosphononorvalinate derivative of formula III are cleaved to give the corresponding (R)-4-oxo-5-phosphononorvaline derivative of formula Ia.

For example, the appropriate (R)-alkyl N-trityl-4-oxo-5-phosphononorvalinate derivative of formula III is treated with two molar equivalents of a suitable trimethylsilyl halide such as trimethylsilyl iodide or trimethylsilyl bromide. The reactants are typically contacted in a suitable organic solvent such as acetonitrile or methylene chloride. The reactants are typically stirred together at room temperature for a period of time ranging from 2-10 hours. The resulting (R)-4-oxo-5-phosphononorvaline derivative of formula Ia is recovered from the reaction zone by extractive methods as is known in the art. It may be purified by chromatography.

In step a₂, the phosphonate methyl ester and carboxylate functionalities of the appropriate (R)-alkyl N-trityl-4-oxo-5-phosphononorvalinate derivative of formula III are cleaved to give the corresponding (R)-4-oxo-5-phosphononorvaline derivative of formula Ib.

For example, the appropriate (R)-alkyl N-trityl-4-oxo-5-phosphononorvalinate derivative of formula III is treated with a suitable strong aqueous mineral acid, such as hydrochloric acid, hydrobromic acid, sulfuric acid and the like. The reactants are typically stirred together for a period of time ranging from 5 minutes to 2 hours and at a temperature range of from room temperature to reflux. The resulting (R)-4-oxo-5-phosphononorvaline derivative of formula Ib is recovered from the reaction zone as its hydrochloride salt by evaporation of the solvent. It may be converted to its free base by treatment with propylene oxide as is known in the art.

In optional step b, the carboxylate functionality of the appropriate (R)-4-oxo-5-phosphononorvaline derivative of formula Ia is cleaved to give the (R)-4-oxo-5-phosphononorvaline derivative of formula Ib as described previously in step a₂.

The following example presents a typical method of using the (R)-alkyl N-trityl-4-oxo-5-phosphononorvalinate derivatives of formula III in order to form the (R)-4-oxo-5-phosphononorvaline derivatives of formula I. This example is understood to be illustrative only and is not intended to limit the scope of the present invention in any way.

### Example 3

### (R)-4-Oxo-5-phosphononorvaline

Add (R)-methyl N-trityl-4-oxo-5-(dimethylphosphono)-norvalinate (1.0g, 2.02mmol) to aqueous 5M hydrochloric acid (10mL) and reflux for 15 hours. Evaporate the resulting solution to give a residue and take the residue up in methanol (8mL). Add propylene oxide (0.5mL) and stir for 0.5 hours. Filter the resulting solid and wash with methanol (5mL). Take the resulting solid up in water (30mL) and freeze dry to give the title compound as a white solid.
¹H NMR (D₂O, 300MHz) 4.3 (m, 1H), 3.48 (m, 2H), 3.12 (m, 2H);
¹³C NMR (D₂O, 300MHz) 208.283, 175.121, 52.161, 52.110, 46.106;
³¹P NMR (D₂O, 121MHz) 12.4;
MS (FAB) m/z 212 (M+H⁺), 167 (M+H⁺ -HCO₂H), 139 (+H₃PO₃CH₂COCH₃), 132 (CH₃COCH₂CH(NH₃+)CO₂H).
Anal. Calcd for C₅H₁₀NO₆P· 0.5H₂O: C, 27.28; H, 5.04; N, 6.45;
Found: C, 27.38; H, 4.77; N, 6.45 (Tg% loss = 4.4).

## Claims

1. A compound of the formula 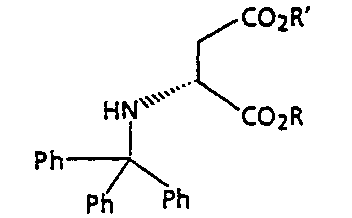 wherein R and R' are simultaneously methyl or ethyl.

2. A compound of the formula 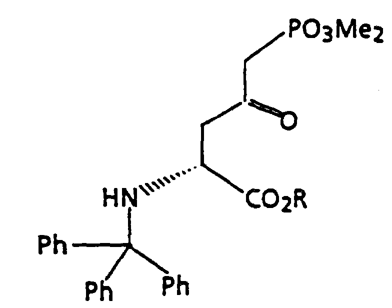 wherein R is methyl or ethyl.

3. A method of using a compound of Claim 1 in order to form a compound of Claim 2 comprising reacting a compound of the Claim 1 with an appropriate metallo dimethyl methylphosphonate.

4. A process for preparing (R)-4-oxo-5-phosphononorvaline ester derivatives comprising reacting compound of Claim 2 with a suitable trimethylsilyl halide.

5. A process according to claim 3 for preparing (R)-4-oxo-5-phosphononorvaline derivatives comprising the steps of:
(a) reacting a compound of the Claim 1 with an appropriate metallo dimethyl methylphosphonate to give a compound of Claim 2;
(b) reacting said compound of Claim 2 with a suitable acid.

6. A process according to claim 3 for preparing (R)-4-oxo-5-phosphononorvaline derivatives comprising the steps of:
a) reacting a compound of Claim 2 with a suitable trimethylsilyl halide to give a (R)-4-oxo-5-phosphononorvaline ester derivative;
b) reacting said (R)-4-oxo-5-phosphononorvaline ester derivative with a suitable acid.

7. A method of using a compound of Claim 2 in order to form (R)-4-oxo-5-phosphononorvaline derivatives comprising reacting said compound of Claim 2 with a suitable acid.

## Patentansprüche

1. Verbindung der Formel 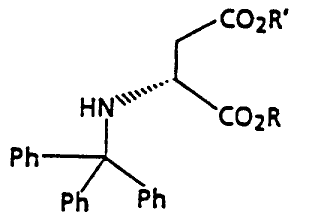 in der R und R' gleichzeitig Methyl- oder Ethylgruppen darstellen.

2. Verbindung der Formel 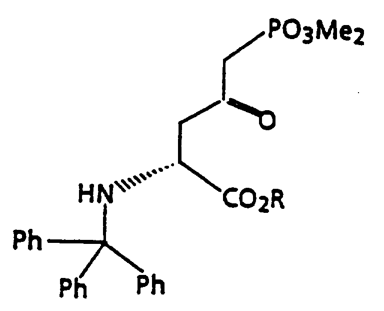 in der R eine Methyl- oder Ethylgruppe ist.

3. Verfahren zur Verwendung einer Verbindung aus Anspruch 1 zur Bildung einer Verbindung nach Anspruch 2, das die Reaktion einer Verbindung nach Anspruch 1 mit einem geeigneten Metallomethanphosphonsäuredimethylester umfaßt.

4. Verfahren zur Herstellung von 4-Oxo-5-phosphono-(R)-norvalinesterderivaten, das die Reaktion einer Verbindung nach Anspruch 2 mit einem geeigneten Trimethylsilylhalogenid umfaßt.

5. Verfahren nach Anspruch 3 zur Herstellung von 4-Oxo-5-phosphono-(R)-norvalinderivaten, das die Schritte umfaßt:
(a) Reaktion einer Verbindung nach Anspruch 1 mit einem geeigneten Metallomethanphosphonsäuredimethylester zu einer Verbindung nach Anspruch 2;
(b) Reaktion dieser Verbindung nach Anspruch 2 mit einer geeigneten Säure.

6. Verfahren nach Anspruch 3 zur Herstellung von 4-Oxo-5-phosphono-(R)-norvalinderivaten, das die Schritte umfaßt:
(a) Reaktion einer Verbindung nach Anspruch 2 mit einem geeigneten Trimethylsilylhalogenid zu einem 4-Oxo-5-phosphono-(R)-norvalinesterderivat;
(b) Reaktion dieses 4-Oxo-5-phosphono-(R)-norvalinesterderivats mit einer geeigneten Säure.

7. Verfahren zur Verwendung einer Verbindung nach Anspruch 2 zur Bildung von 4-Oxo-5-phosphono-(R)-norvalinderivaten, das die Reaktion einer Verbindung nach Anspruch 2 mit einer geeigneten Säure umfaßt.

## Revendications

1. Composé de formule 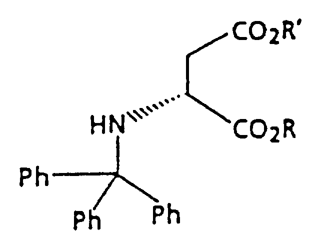 dans laquelle R et R' représentent simultanémént un groupe méthyle ou éthyle.

2. Composé de formule 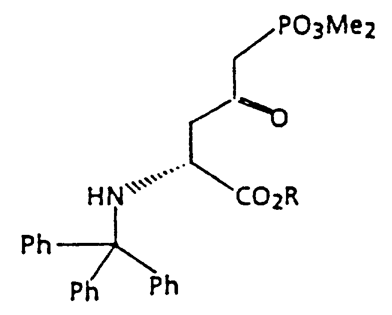 dans laquelle R représente un groupe méthyle ou éthyle.

3. Procédé d'utilisation d'un composé de la revendication 1, dans le but de former un composé de la revendication 2, comprenant la réaction d'un composé de la revendication 1 avec un méthylphosphonate de diméthyle métallique approprié.

4. Procédé de préparation de dérivés d'ester de (R)-4-oxo-5-phosphononorvaline, comprenant la réaction d'un composé de la revendication 2 avec un halogénure de triméthylsilyle approprié.

5. Procédé selon la revendication 3, permettant de préparer des dérivés de (R)-4-oxo-5-phosphononorvaline comprenant les étapes consistant à :
(a) faire réagir un composé de la revendication 1 avec un méthylphosphonate de diméthyle métallique approprié afin d'obtenir un composé de la revendication 2 ;
(b) faire réagir ledit composé de la revendication 2 avec un acide approprié.

6. Procédé selon la revendication 3, permettant de préparer des dérivés de (R)-4-oxo-5-phosphononorvaline comprenant les étapes consistant à:
a) faire réagir un composé de la revendication 2 avec un halogénure de triméthylsilyle approprié pour obtenir un dérivé d'ester de (R)-4-oxo-5-phosphononorvaline ;
b) faire réagir ledit dérivé d'ester de (R)-4-oxo-5-phosphononorvaline avec un acide approprié.

7. Procédé d'utilisation d'un composé de la revendication 2, destiné à former des dérivés de (R)-4-oxo-5-phosphononorvaline comprenant la réaction dudit composé de la revendication 2 avec un acide approprié.
